Europäisches Patentamt

European Patent Office

Office européen des brevets

(19)

(11) Publication number: **0 027 726**
**B1**

(12) **EUROPEAN PATENT SPECIFICATION**

(45) Date of publication of patent specification: **11.07.84**

(51) Int. Cl.³: **A 61 B 17/18**

(21) Application number: **80303671.4**

(22) Date of filing: **17.10.80**

(54) Improved orthopedic fixation pin holder.

(30) Priority: **18.10.79 US 85996**
**22.10.79 US 87218**

(43) Date of publication of application:
**29.04.81 Bulletin 81/17**

(45) Publication of the grant of the patent:
**11.07.84 Bulletin 84/28**

(84) Designated Contracting States:
**BE DE FR GB IT NL SE**

(56) References cited:
**US - A - 1 997 466**
**US - A - 2 393 694**

(73) Proprietor: **ACE ORTHOPEDIC MANUFACTURING, INC.**
**11913 S. Prarie Avenue**
**Hawthorne, California 90250 (US)**

(72) Inventor: **Dohogne, Charles L.**
**29615 Enrose Avenue**
**San Pedro California 90732 (US)**

(74) Representative: **Jones, Colin et al,**
**W.P. THOMPSON & CO. Coopers Building Church Street**
**Liverpool L1 3AB (GB)**

Courier Press, Leamington Spa, England.

## Description

The present invention relates to medical pin holder devices and more particularly to medical pin holder devices utilized in orthopaedic external fixation apparatus wherein the repositioning and immobilization of a fractured bone is facilitated by means external of the soft tissue surrounding the fractured bone.

Various external fixation devices are currently available in the market-place, all of which in one form or another utilize plural transfixing and/or half pins positioned on opposite sides of a bone fracture which extend through the fractured bone and outward beyond the soft tissue surrounding the bone. The exposed ends of the plural pins are rigidly attached to one or more pin holders which are interconnected by multiple adjustment rods to form an external frame about the soft tissue of a patient. By adjusting the relative orientation of the pin holders on opposite sides of the fracture and securely maintaining a desired orientation during rehabilitation, the bone fracture may be accurately realigned to permit proper healing of the fracture. Thus, the pin holders form an integral component of the entire orthopaedic external fixation device which must be capable of rigidly clamping the pins in a desired position to provide complete immobilization of the bone fracture.

Heretofore, the prior art pin holder devices utilized in orthopaedic external fixation apparatus have been formed in various design configurations, all of which have provided for the selective clamping of either singular or multiple pins within the pin holder. Such a pin-holder is described e.g. in US—A—2 393 694. Although such prior art pin holder devices have proven useful in general applications, there are inherent deficiencies associated in their use.

These inherent deficiencies have focused primarily upon the prior art pin holder's inability to provide sufficient clamping forces to positively prevent movement of the pins within the pin holder as well as a failure to provide any means for the independent removal or adjustment of individual pins upon the pin holder. As such, the prior art devices have often permitted the undesirable movement of the fractured bone during rehabilitation or limited operative and post-operative adjustment and modification of the pins upon the patient.

Thus, there exists a substantial need for an improved external fixation pin holder device which rigidly clamps the pin in a desired position, and additionally provides for the independent adjustment of multiple pins within the pin holder.

The present invention resides in a pin holder device for use on an orthopaedic external fixation device comprising a body member adapted to be mounted to said external fixation device, an aperture extending through said body member and sized to receive a pin therein, characterised in that a second aperture extends through said body member intersecting and oriented perpendicular to said first aperture, the second aperture including an enlarged aperture portion initiating at one end of said second aperture and terminating at a distance spaced from the opposite end of said second aperture, and in that a locking member is slidingly positioned within said enlarged aperture portion and has a pin-receiving aperture oriented parallel to said first aperture, said locking member including means for displacing said locking member within said enlarged aperture portion to cause said pin receiving aperture and said first aperture to tightly contact opposite sides of a pin inserted through said apertures; and further characterised in that a V-shaped recess is formed on opposing wall portions of said pin-receiving aperture and said first aperture, said recesses tangentially contacting said pin at discrete locations along the periphery of said pin to apply a shear force to said pin to positively clamp said pin in said pin holder.

Thus, individual pins can be positively clamped within a common pin holder and independent adjustment of a desired pin without disturbing the remaining pins within the pin holder is facilitated.

The present invention will be further described, by way of example, with reference to the drawings wherein:

Figure 1 is a partially exploded perspective view of a pin holder according to the present invention, illustrating its detailed construction and depicting a manner in which it may be mounted to a support frame of an external fixation device;

Figure 2 is a cross-sectional view of a portion of the pin holder of Figure 1, taken on lines 2—2 of Figure 1, showing the shape and orientation of a pocket formed therein which receives the pin lock member of Figure 1; and

Figure 3 is a larger scale detail side view of the pin holder of Figure 1, illustrating the manner in which an individual pin is rigidly locked in place within the pin holder.

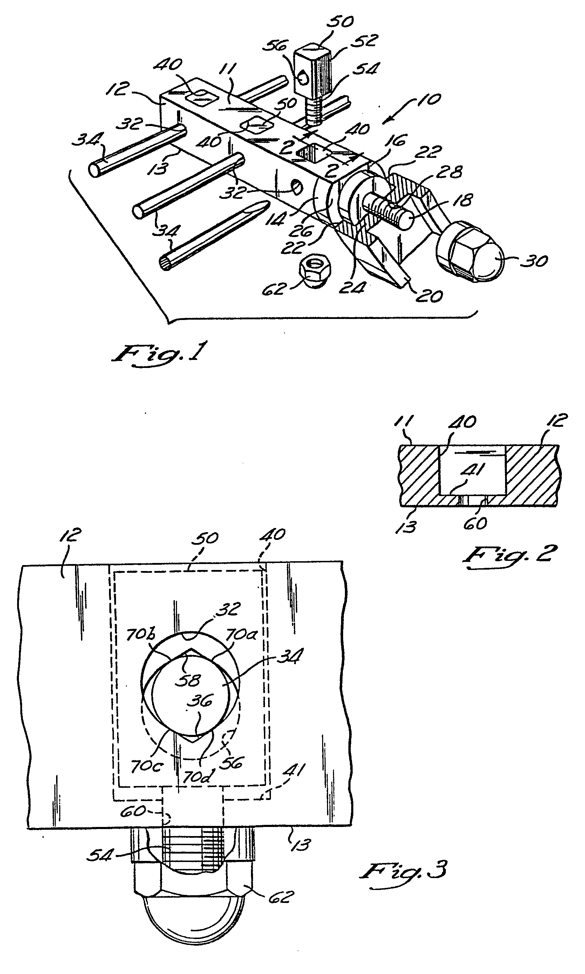

Referring to Figure 1, an improved pin holder 10 according to the present invention is specifically adapted for use upon a particular orthopaedic external fixation device.

As shown in Figure 1, the pin holder 10 of the present invention has an elongate body section 12 including a flange 14 at its proximal end. The flange 14 is provided with a mounting boss 16 and a threaded stud 18 which are adapted to mount the pin holder 10 to an I-beam frame segment 20 of the external fixation device (not shown). The length of the mounting boss 16 is slightly less than the distance between the outboard edges 22 and web portion 24 of the frame segment 20, such that the lower surface 26 of the flange 14 may abut the outboard edges 22 of the frame segment 20. With the threaded stud 18 inserted through a mounting aperture 28 formed in the frame segment 20, an acorn fastener 30 may be threaded onto the stud 18, from the opposite side of the frame segment 12, thereby rigidly mounting the pin holder 10 to the frame segment.

By such an arrangement, the pin holder 10 may be selectively rotated about the axis of the threaded stud 18 and subsequently locked in a desired orientation by the manual tightening of the acorn fastener 30 againt the web portion 24 of the frame segment 12. Further, it will be recognized that the mounting boss 16 and threaded stud 18 may be modified with other conventional mounting means to permit the pin holder 10 of the present invention to be utilized on other particular types of external fixation devices (not shown).

The body section 12 of the pin holder 10 includes a plurality of apertures 32 extending through its width, each sized to loosely receive the exposed end of a transfixing or half pin 34 which extends through the body's soft tissue (not shown) and into the fractured bone (not shown). As best shown in Figure 3, the lower portion of each of the apertures 32 is formed with a V-shaped wall portion 36 having an included angle of approximately 120 degrees.

A plurality of rectangular pockets 40 are formed in the body section 12 of the pin holder 10 being disposed perpendicular to the apertures 32. The pockets 40 initiate at the upper surface 11 of the body section 12 terminating at a distance below the apertures 32 but above the lower surface 13 of the body section 12 (shown in Figure 2). Pockets of any other non-circular shape may, of course, be used.

Each of the pockets 40 is sized to slidingly receive a pin lock member 50 having a rectangular shaped body 52 and a threaded stud 54. The body 52 of the pin lock member 50 includes a central aperture 56 which extends through the pin lock member 50 and is provided with a V-shaped wall portion 58 adjacent its upper end, which is formed in the manner previously described in relation to the V-shaped wall 36 of the apertures 32. The threaded stud 54 of the pin lock member 50 extends through an aperture 60 (see Figure 2) formed centrally between the lower surface 41 of the pocket 40 and the bottom surface 13 of the body section 12, and receives an acorn fastener 62.

The use of the pin holder 10 of the present invention may be described with specific reference to Figures 1 and 3. In use, the pin lock member 50 is loosely positioned within one of the pockets 40 formed in the body section 12, and the exposed end of the transfixing or half pin 34 is inserted through both of the apertures 32 and 56 formed in the body section 12 and pin lock member 50, respectively. Subsequently, the acorn fastener 62 may be mounted onto the threaded stud 54 causing the pin lock member 50 to be pulled tightly downward toward the lower surface 41 of the pocket 40.

During this downward movement of the pin lock member 50, the horizontal center line of the aperture 56 formed in the pin lock member 50 passes beneath the horizontal center line of the aperture 32 formed in the body section 12, whereby the opposed V-shaped wall portions 36 and 58 contact the outside diameter of the pin 34. Due to the V-shaped configuration of the wall portions 36 and 58, upon contact therewith, the pin 34 is self-centered along the aligned vertical center lines of the apertures 32 and 56.

Continued manual tightening of the acorn fastener 62 causes the pin 34 to be tightly clamped between the opposing V-shaped wall portions 36 and 58 with the clamping forces being concentrated along four discrete clamping lines 70a, 70b, 70c and 70d corresponding to the tangential contact between the outer diameter of the pin 34 with the V-shaped wall portions 36 and 58. The application of the concentrated clamping forces along the four discrete clamping lines 70a to 70d generates a shearing force upon the pin 34 which has been found to positively clamp the pin 34 within the pin holder 14.

Additionally, as shown in Figure 1, each of the pockets 40 formed in the body section 12 of the pin holder 14 is provided with a respective pin lock member 50 which facilitates multiple pins 34 to be positively clamped within the pin holder 10. Due to each of the pins 34 being mounted to the pin holder 10 by use of a separate pin lock member 50, individual pins may be selectively adjusted within the pin holder 10 by merely loosening the appropriate acorn fastener 62 from the respective pin lock member 50 without disturbing or affecting the other pins 34 in the holder 10. As such, the pin holder 10 permits an orthopaedic surgeon to independently adjust and modify pin fixation upon the patient to meet necessary operative and post-operative procedures.

Thus, in summary, the pin holder 10 of the present invention, by use of the novel V-shaped wall portions 36 and 58 and individual pin lock members 50 yields independent positive clamping of multiple pins 34 upon an external fixation device. Although in the preferred embodiment the pin holder 14 is formed to clamp three individual pins 34, those skilled in the art will recognize that the present invention is equally applicable to single as well as multiple pin clamping operations. Also, while the description refers to certain orientation as upper and lower, it will be evident that this is only for convenience and description and does not limit the orientation of the pin holder.

## Claims

1. A pin holder for use on an orthopaedic external fixation device comprising a body member (12) adapted to be mounted to said external fixation device, an aperture (32) extending through said body member and sized to receive a pin (34) therein, characterised in that a second aperture (40, 60) extends through said body member (12) intersecting and oriented perpendicular to said first aperture (32), the second aperture including an enlarged aperture portion (40) initiating at one end of said second aperture and terminating at a distance spaced from the opposite end of said second aperture, and in that a locking member (50) is slidingly positioned within said enlarged aperture portion and has a pin-receiving aperture (56) oriented parallel to said first aperture (32), said locking member including means (54, 62) for displacing said locking member within said enlarged aperture portion to cause said pin receiving aperture (56) and said first aperture (32) to tightly contact opposite sides of a pin (34) inserted through said apertures; and further in that a V-shaped recess (70) is formed on opposing wall portions of said pin-receiving aperture (56) and said first aperture (32), said recesses (70) tangentially contacting said pin (34) at discrete locations along the periphery of said pin to apply a shear force to said pin to positively clamp said pin in said pin holder (10).

2. A pin holder as claimed in claim 1, in which the body member (12) has several apertures (32) for receiving a corresponding number of pins (34) therein, and a separate locking member (50) in a corresponding aperture (40, 60) for each pin.

## Revendications

1. Support de broches destiné à être utilisé sur un dispositif de fixation externe orthopédique, comprenant un corps (12) conçu pour être monté sur ledit dispositif de fixation externe, une ouverture (32) traversant ledit corps et dimensionnée pour recevoir une broche (34), caractérisé en ce qu'une seconde ouverture (40, 60) traverse ledit corps (12), coupant ladite première ouverture (32) à laquelle elle est orientée perpendiculairement, la seconde ouverture comprenant une partie d'ouverture élargie (40) commençant à une première extrémité de ladite seconde ouverture et se terminant à une certaine distance de l'extrémité opposée de ladite seconde ouverture, et en ce qu'un élément (50) de verrouillage est glissé en position à l'intérieur de ladite partie d'ouverture élargie et présente une ouverture (56) de réception d'une broche orientée parallèlement à ladite première ouverture (32), ledit élément de verrouillage comportant des moyens (54, 62) permettant de déplacer ledit élément de verrouillage à l'intérieur de ladite partie d'ouverture élargie pour que ladite ouverture (56) de réception d'une broche et ladite première ouverture (32) portent étroitement contre des côtés opposés d'une broche (34) introduite dans lesdites ouvertures; et en outre en ce qu'un évidement (70) de forme en V est formé sur des parties de parois opposées de ladite ouverture (56) de réception d'une broche et de ladite première ouverture (32), lesdits évidements (70) portant tangentiellement contre ladite broche (34) en des points physiquement distincts de la périphérie de ladite broche afin d'appliquer une force de cisaillement à ladite broche pour brider fermement ladite broche dans ledit support (10) de broche.

2. Support de broches selon la revendication 1, dans lequel le corps (12) présente plusieurs ouvertures (32) destinées à recevoir un nombre correspondant de broches (34), et un élément séparé (50) de verrouillage disposé dans une ouverture correspondante (40, 60) pour chaque broche.

## Patentansprüche

1. Stifthalter zur Verwendung an einer äusseren orthopädischen Fixierungsvorrichtung, mit einem Hauptteil (12), der zur Befestigung an der äusseren Fixierungsvorrichtung ausgebildet ist, und mit einer Öffnung (32), die sich durch den Hauptteil hindruch erstreckt und in ihrer Grösse so bemessen ist, dass sie in sich einen Stift (34) aufnehmen kann, dadurch gekennzeichnet, dass eine zweite Öffnung (40, 60) sich durch den Hauptteil (12) hindurch erstreckt und die erste Öffnung (32) schneidet und senkrecht zu dieser gerichtet ist, dass die zweite Öffnung einen vergrösserten Öffnungsabschnitt (40) aufweist, der an einem Ende der zweiten Öffnung beginnt und in einem Abstand von dem gegenüberliegenden Ende der zweiten Öffnung endet, und das ein Verriegelungsglied (50) gleitend innerhalb des vergrösserten Öffnungsabschnitts angeordnet ist und eine Stiftaufnahmeöffnung (56) aufweist, die parallel zu der ersten Öffnung (32) orientiert ist, wobei das Verriegelungsglied Mittel (54, 62) zum Verschieben des Verriegelungsglieds innerhalb des vergrösserten Öffnungsabschnitts aufweist, um die Stiftaufnahmeöffnung (56) und die erste Öffnung (32) zu veranlassen, sich dicht an gegenüberliegende Seiten eines Stifts (34) anzulegen, der durch diese Öffnungen eingesetzt ist, und dass ferner eine V-förmige Ausnehmung (70) an gegenüberliegenden Wandabschnitten der Stiftaufnahmeöffnung (56) und der ersten Öffnung (32) gebildet ist, wobei diese Ausnehmung (70) den Stift (34) tangential an bestimmten Stellen längs des Umfangs des Stifts berührt, um eine Scherkraft auf den Stift aufzubringen und den Stift positiv in dem Stifthalter (10) festzuklemmen.

2. Stifthalter nach Anspruch 1, dadurch gekennzeichnet, dass der Hauptteil (12) mehrere Öffnungen (32) zur Aufnahme einer entsprechenden Anzahl von Stiften (34) aufweist und dass ein getrenntes Verriegelungsglied (50) in einer entsprechenden Öffnung (40, 60) für jeden Stift vorgesehen ist.

Fig.1

Fig.2

Fig.3

1